(19) **Europäisches Patentamt / European Patent Office / Office européen des brevets**

(11) **EP 4 699 527 A1**

(12) **EUROPEAN PATENT APPLICATION**

(43) Date of publication:
**25.02.2026 Bulletin 2026/09**

(21) Application number: **24382920.7**

(22) Date of filing: **20.08.2024**

(51) International Patent Classification (IPC):
**A61B 5/05** (2021.01)    **A61B 5/0507** (2021.01)
**A61B 5/06** (2006.01)    **A61B 8/00** (2006.01)
**A61F 2/82** (2013.01)    **H01P 3/02** (2006.01)

(52) Cooperative Patent Classification (CPC):
**A61B 5/0507; A61B 5/05; A61B 5/06; A61B 5/062;**
A61F 2/82

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Nimble Diagnostics, S.L.**
**08916 Badalona Barcelona (ES)**

(72) Inventors:
• **O'CALLAGHAN CASTELLA, Joan**
  **08916 Badalona (ES)**
• **AMORÓS GARCÍA DE VALDECASAS, Susana**
  **08916 Badalona (ES)**
• **IBORRA EGEA, Oriol**
  **08916 Badalona (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **STENT MONITORING SYSTEM AND METHOD THEREOF**

(57)    The present disclosure relates to a method and a system designed to monitor the integrity of one or more stents using their electrical properties, in particular their ability to scatter electromagnetic fields, and the relationship of said scattering with the integrity of the stent. The system comprises one or more antennas and a measuring device that communicates with these antennas. The measuring device is configured to obtain frequency and/or time responses of electromagnetic fields such as those scattered by the stent and those scattered by a reference scatterer, and/or those directly transmitted between antennas. The system further processes these responses to determine representative frequencies and echo delays of said responses, which are then used to compute a mathematical relationship indicative of the stent's integrity.

## Description

## Technical field of the invention

This present invention belongs to the medical field, particularly to the field of stent monitoring. More particularly, the present invention refers to a system to monitor the integrity of stents and to a method thereof.

## Background of the invention

A stent is an endovascular prosthesis inserted into the inside space of an anatomic vessel or duct to keep the passageway open. A stent may comprise a plastic or metallic cylindrical tubular body which is expanded by means of an inflatable balloon catheter in order to fix it to the vessel walls. It is well known its use for the treatment of some coronary, vascular and biliary diseases by restoring partial occlusions, also known as stenosis. Stents are left fixed to the vessel wall to prevent re-occlusion, also known as restenosis, and when a stent is used and restenosis still occurs, this is called in-stent restenosis or ISR.

Therefore, patients with stents may require monitoring to prevent ISR and/or deterioration of the stent structural integrity. Some conditions are well-known to be directly associated with stent failure, including in-stent neointimal proliferation, stent fracture, misplacement, thrombosis and in-stent neo-atherosclerosis.

Identification of these high-risk conditions may require invasive and complex procedures such as coronary angiography, intravascular ultrasound or optical coherence tomography, which are invasive techniques requiring hospital admission of the patient, are expensive and need trained physicians. Besides, in some cases the use of specifically configured stents may be necessary. This specific configuration may comprise the use of additional apparatus attached to the stent, such as RFID tags, or the use of specific materials and/or shapes. Eventually, that means a higher cost and a lower number of possible patients that can be monitored with such techniques.

It is also known the use of electromagnetic microwave spectrometry to detect structural damage in stents by determining the resonance frequencies of stents in air and water. In this case the stent is rotating and is placed symmetrically between two antennas, which are difficult conditions, if not impossible, to be replicated in live patients. Furthermore, near-field electromagnetic microwave probes have been used for stent monitoring in-vivo in mice. However, they were only able to couple with non-distant stents, below 1 cm, and therefore they are unable to monitor deep prosthesis such as coronary stents, which are usually found at a distance of approximately 3 cm from the skin surface..

Other antennas different than near-field antennas, on the other hand, allow the receiver antenna to receive direct electromagnetic radiation from the emitter antenna, and in consequence show a perturbation in the transmission coefficient in the presence of a stent, due to the interference between the direct electromagnetic field and the scattered electromagnetic field by the stent. However, this perturbation cannot be directly used to monitor the stent since it occurs at a frequency that depends on the difference between the length of the propagation paths of the direct signal between the antennas, and the scattered signal by the stent, which is unknown in pre-clinical or clinical trials.

It is therefore needed a non-invasive system to monitor the integrity of a stent placed in a vessel or duct of a patient, in order to obtain information related to structural damage, malposition, thrombosis and/or in-stent restenosis, among other conditions. It should work at depths above 1 cm, therefore allowing monitoring deep prosthesis such as coronary stents, and should not depend on the length of the propagation path. Furthermore, it must be possible to use it in vivo. Finally, said integrity should be quantified in order to follow the evolution of the treatment.

## Summary of the invention

According to a first aspect of the invention, a system configured to monitor the integrity of a stent is disclosed. The system comprises one or more antennas and a measuring device in communication with the one or more antennas. The measuring device is configured to obtain a frequency response and/or a time response of a first scattered electromagnetic field launched from at least one emitter antenna, scattered by a reference scatterer different than the stent, and collected by the same at least one emitter antenna or, in case the system comprises at least two antennas, collected by a different at least one receiver antenna. Instead, or in addition, the measuring device is configured to obtain a frequency response and/or a time response of a direct electromagnetic field launched from at least one emitter antenna and directly collected by a different at least one receiver antenna in case the system comprises at least two antennas.

The measuring device is further configured to obtain a frequency response and/or a time response of a second scattered electromagnetic field launched from at least one emitter antenna, scattered by the stent, and collected by the same emitter antenna or by a different at least one receiver antenna in case the system comprises at least two antennas. The measuring device determines, from the frequency response and/or the time response of the first scattered electromagnetic field, a representative frequency and an echo delay associated with the first scattered electromagnetic field, and/or determines from the frequency response and/or time response of the direct electromagnetic field a representative frequency and an echo delay associated with the direct electromagnetic field.

The measuring device also determines, from the frequency response and/or the time response of the second scattered electromagnetic field, a representative frequency and an echo delay associated with the second scattered electromagnetic field. It then computes a mathematical relationship ($m_D$) between the representative frequency and/or the echo delay associated with the first scattered electromagnetic field, and/or between the representative frequency and/or the echo delay associated with the direct electromagnetic field. Additionally, the measuring device computes a mathematical relationship ($m_S$) between the representative frequency and/or the echo delay associated with the second scattered electromagnetic field component.

Finally, the measuring device computes a mathematical relationship ($I_S$) between the parameter mo and the parameter ms, preferably wherein Is comprises a ratio between mo and $m_S$. The mathematical relationship $I_S$ is indicative of the integrity of the stent. In some embodiments $I_S$ may be compared with reference measurements, previous measurements, tabulated values, and/or mathematical models, among other options, to determine the integrity of the stent.

In a preferred embodiment of the first aspect of the invention, in the case that the system comprises two or more antennas, an emitter antenna is configured to launch an electromagnetic field onto a stent, such that the stent scatters the impinging electromagnetic field. A receiver antenna, different from the emitter antenna, is configured to collect electromagnetic fields, wherein the collected electromagnetic fields comprise direct electromagnetic fields emitted from the emitter antenna and directly collected by the receiver antenna, as well as scattered electromagnetic fields emitted from the emitter antenna, scattered from the stent, and collected by the receiver antenna.

The measuring device is further configured to obtain the frequency response and/or the time response of the collected electromagnetic fields in the receiver antenna by measuring an input signal of the emitter antenna and measuring an output signal of the receiver antenna. The input signal is related to the electromagnetic field launched onto the stent by the emitter antenna, and the output signal is related to the collected electromagnetic fields in the receiver antenna. The system then obtains a scattering coefficient ($S_T$) between the emitter antenna and the receiver antenna by comparing the input signal with the output signal. Finally, the system obtains a frequency response S(f) of the scattering coefficient ($S_T$) and/or a time response S(t) of the scattering coefficient ($S_T$).

In a more preferred embodiment of the first aspect of the invention, the emitter antenna is configured to collect electromagnetic fields comprising scattered electromagnetic fields from the stent. The measuring device is further configured to obtain the frequency response and/or the time response of the collected electromagnetic fields in the emitter antenna by measuring an input signal of the emitter antenna and an output signal of the emitter antenna. The input signal is related to the electromagnetic field launched onto the stent by the emitter antenna, and the output signal is related to the collected electromagnetic fields in the emitter antenna. The system then obtains a scattering coefficient ($S_R$) by comparing the input signal with the output signal of the emitter antenna. Finally, the system obtains a frequency response R(f) of the scattering coefficient ($S_R$) and/or a time response R(t) of the scattering coefficient ($S_R$).

In another preferred embodiment of the first aspect of the invention, the system comprises at least two antennas in communication with the measuring device. The measuring device is configured to obtain a frequency response and/or a time response of a direct electromagnetic field launched from at least one emitter antenna and directly collected by a different at least one receiver antenna. It is also configured to obtain a frequency response and/or a time response of a scattered electromagnetic field launched from at least one emitter antenna, scattered by the stent, and collected by the same emitter antenna.

The measuring device further determines from the frequency response and/or time response of the direct electromagnetic field a representative frequency and an echo delay associated with the direct electromagnetic field. Additionally, it determines from the frequency response and/or the time response of the scattered electromagnetic field a representative frequency and an echo delay associated with the scattered electromagnetic field. The system then computes a mathematical relationship ($m_D$) between the representative frequency and/or the echo delay associated with the direct electromagnetic field, and a mathematical relationship (ms) between the representative frequency and/or the echo delay associated with the scattered electromagnetic field component. Finally, the system computes a mathematical relationship (IS) between the parameter mo and the parameter ms, preferably where IS comprises a ratio between mo and ms, wherein the mathematical relationship IS is indicative of the integrity of the stent.

In another preferred embodiment of the first aspect of the invention, obtaining the frequency response of a collected electromagnetic field comprises launching a fixed-frequency electromagnetic field from the emitter antenna for a period of time T and for two or more frequencies comprised in a range of frequencies R, or launching a frequency-modulated electromagnetic field from the emitter antenna for a period of time T', wherein the frequency-modulated electromagnetic field continuously varies over the frequencies comprised in a frequency range R', or launching an electromagnetic field having a spreading modulation from the emitter antenna for a period of time T", wherein the spreading-modulated electromagnetic field covers a frequency range R", and measuring the collected electromagnetic fields in the emitter and/or in the receiver antenna.

In a preferred embodiment of the first aspect of the invention, performing the time response of a collected electromagnetic field comprises launching an ultra wide-band (UWB) electromagnetic impulse from the emitter antenna for a period of time T", wherein the UWB impulse comprises the frequencies comprised in the frequency range R", and measuring the collected electromagnetic fields in the emitter and/or the receiver antenna.

In another preferred embodiment of the first aspect of the invention, the representative frequencies associated with the direct and first and second scattered electromagnetic fields are the weighted relative average of the frequencies of said electromagnetic fields, wherein the frequencies are averaged according to the corresponding relative amplitude between the emitted and received electromagnetic fields.

In a more preferred embodiment of the first aspect of the invention, the scattering coefficient (ST) is obtained as a complex ratio between the electromagnetic field launched from the emitter antenna and the electromagnetic field collected in the receiver antenna, and the scattering coefficient ($S_R$) is obtained as a complex ratio between the electromagnetic field launched from the emitter antenna and the electromagnetic field collected in the same emitter antenna.

According to a preferred embodiment of the first aspect of the invention, the mathematical relationship (IS) is computed as or proportional to

$$I_S(\%) = \left(1 - \frac{m_D}{m_S}\right)$$
.

In another preferred embodiment of the first aspect of the invention, all the frequency and/or time response measurements are normalized by being divided by an equalizing scattering coefficient.

In a further preferred embodiment of the first aspect of the invention, the measuring device is configured to measure the input and output signals in synchrony with the heartbeat of the subject comprising the stent, preferably by using a transformed ECG signal as a trigger signal wherein this trigger signal is provided to the measuring device to launch the electromagnetic fields. More preferably, the system further comprises means for obtaining the ECG of the subject, and the measuring device is configured to receive the ECG signal from the means for obtaining the ECG.

According to a preferred embodiment of the first aspect of the invention, the antennas are co-polarized antennas.

In a further preferred embodiment of the first aspect of the invention, the antennas are linearly polarized at a frequency span that includes the average stent resonance frequency in biological tissue (0.7 GHz), preferably the span ranges between 0.1 and 3 GHz.

In another preferred embodiment of the first aspect of the invention, mD comprises or consists of the representative frequency times the echo delay of the direct electromagnetic field launched from an emitter antenna and directly collected by a receiver antenna different than the emitter antenna, and mS comprises or consists of the representative frequency times the echo delay of the scattered electromagnetic field component launched from an emitter antenna, scattered by the stent and collected by a receiver antenna different than the emitter antenna. Optionally, the parameter mS further comprises or is averaged with the representative frequency of the electromagnetic field launched from an emitter antenna, reflected and/or scattered by the stent and collected by the same antenna.

In a preferred embodiment of the first aspect of the invention, mS comprises a representative frequency and/or an echo delay associated with a simulation or in vitro measurements of different stent and/or lesion models, in addition to, or instead of the second scattered electromagnetic field component.

## Brief description of the drawings

To enable a better understanding of the present disclosure, and to show how the present disclosure may be carried out, reference will now be made, by way of example only, to the accompanying schematic drawings, wherein:

Figure 1 shows a diagram of two antennas monitoring a stent according to one or more embodiments of the invention.

Figure 2 also shows a diagram of two antennas monitoring a stent according to one or more embodiments of the invention.

Figure 3 shows a diagram of a system to monitor stents according to one or more embodiments of the invention.

Figure 4 shows a diagram of an Extended Gap Ridge Horn (EGRH) antenna according to one or more embodiments of the invention.

Figure 5 shows a set of graphs of measured and fitted transmission coefficient signals corresponding to direct and scattered electromagnetic fields, in the presence of a stent and in the frequency and time domains in a two antennae configuration, according to one or more embodiments of the invention.

Figure 6 shows a set of graphs of measured and fitted transmission coefficient signals corresponding to direct and scattered electromagnetic fields, without the presence of a stent, and in the frequency and time domains in a two antennae configuration, according to one or more embodiments of the invention.

Figure 7 shows a set of tables with all the obtained values of reference frequencies ($f_0$) and echo delays

($t_0$) for different days and different pigs during a first in-vivo experiment after stent implantation, according to one or more embodiments of the invention.

Figure 8 shows a comparison between the stent integrity I (%) measured by histological data and the stent monitoring system of the invention, during the first in-vivo experiment according to one or more embodiments of the invention.

## Description of the invention

## Definitions

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of' excludes any element, step, or ingredient not specified in the claim element. When used herein, "consisting essentially of" does not exclude materials or steps that do not materially affect the basic and novel characteristics of the claim.

The term "integrity", in the context of the invention, refers the level of integrity of a stent that has been placed inside a duct or vessel of a patient. Therefore, the integrity of a stent is compromised when said stent suffers any type of structural damage, becomes mispositioned, its lumen is reduced by any means, possibly leading to thrombosis, or if the vessel or duct of the patient where the stent is placed suffers any type of in-stent restenosis, including asymmetrical in-stent restenosis. The integrity of the stent is preserved when this value is the same than a reference value, generally obtained at the moment of placing the stent on the patient or soon after. If measured in percentage, the integrity is preserved when the value is 100%. The deviation from the 100% in damaged stents may be determined by using other reference values of known damaged stents with different degrees of damage and/or by using reference values of the medium in which the stent is placed, among other options.

The term "stent", in the context of the invention, refers to a tubular metallic or metal-coated device which is inserted into the lumen of an anatomic vessel or duct to keep the passageway open. It may be used to treat coronary, vascular, biliary or urine related diseases, and may be produced from a biocompatible material such as an alloy wire or from a biodegradable material.

The term "antenna" refers to a metallic structure, or a non-metallic structure covered by a thin metallic layer, such as metallic paint, that captures and/or transmits electromagnetic waves. It is a transducer that converts guided to unguided electromagnetic waves or vice versa. In the context of the present invention an antenna can both emit and receive electromagnetic radiation emitted from other antennas in the system.

The term "restenosis" refers to the recurrence of abnormal narrowing of an anatomic vessel or duct after corrective surgery. It may occur when an artery that was opened with a stent or angioplasty becomes narrowed again. When it happens with the stent still inside the vessel it is called "in-stent restenosis", and, in the context of this invention, it may involve any type of structural damage on the stent.

The term "transmission coefficient", refers to any of the non-diagonal parameters in a scattering matrix that quantifies how electromagnetic energy propagate through a multiport network. In the context of this invention, there are at least two ports (antennas) and therefore a corresponding scattering matrix is at least a (2x2) matrix with two transmission coefficients, $S_{12}$ and $S_{21}$ which relate, respectively, to the signal measured in antenna 2 when antenna 1 is excited and the signal measured in antenna 1 when antenna 2 is excited. However, the scattering matrix may be greater (e.g. a 3x3, 4x4, 5x5, ... matrix) and therefore there may be more transmission coefficients. From herein after, reference may be made, just as an example, to a two-antenna configuration with a 2x2 matrix, although it is understood that the same analysis may be made to configurations with more antennas and transmission coefficients, as the skilled person will appreciate. It is noted that in the context of this invention, the term "transmission coefficient" may refer to $S_{12}$, $S_{21}$, both or an average of both indistinctly, since all devices and materials involved are reciprocal. The scattering matrix parameters are complex numbers and can be presented in linear magnitude or logarithmic based decibels, preferably in logarithmic based decibels.

The term "direct electromagnetic field", in the context of this invention, refers to the electromagnetic field received in an antenna coming directly from another antenna, without being scattered by a stent.

The term "scattered electromagnetic field" in the context of this invention, refers to the electromagnetic field received in an antenna after a scatterer, such as a stent, a reference scatterer or the transmission medium scatters the signal, wherein said signal may be emitted from that same antenna or from another antenna.

The term "frequency response" of a "scattered electromagnetic field" refers to a measurement of the scattered electromagnetic field as a function of frequency for a given range of frequencies. The frequency response of a scattered electromagnetic field may comprise the frequency response of a transmission coefficient in a system such as the one from the invention.

The term "reference frequency", in the context of this invention, refers to the frequency at which electric energy stored in an object equals stored magnetic energy resulting in a relative maximum or minimum of the transmission coefficient.

The term "echo delay" in the context of this invention is defined as the time between the emission and the reception of a direct electromagnetic field or an scattered electromagnetic field. Echo delay depends on the propagation paths of the direct and scattered electromagnetic fields and on the properties of the propagation medium, and in the case of scattered signals, also on the properties of the object producing the scattering, such as a stent or a reference scatterer.

The term "frequency domain" refers to the analysis of mathematical functions or signals with respect to frequency.

The term "time domain" refers to how a signal changes over time, whereas a "frequency domain" graph shows how much of the signal is present among each given frequency band.

The term "co-polarised", in the context of this invention, refers to two or more antennas comprising an equivalent polarization, such that radiation emitted from one of them can be received by the other one and vice-versa.

The term "dielectric biomimetic filling", in the context of this invention, refers to a material that emulates the dielectric properties (electrical permittivity and conductivity) of a biological tissue.

## Description

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited.

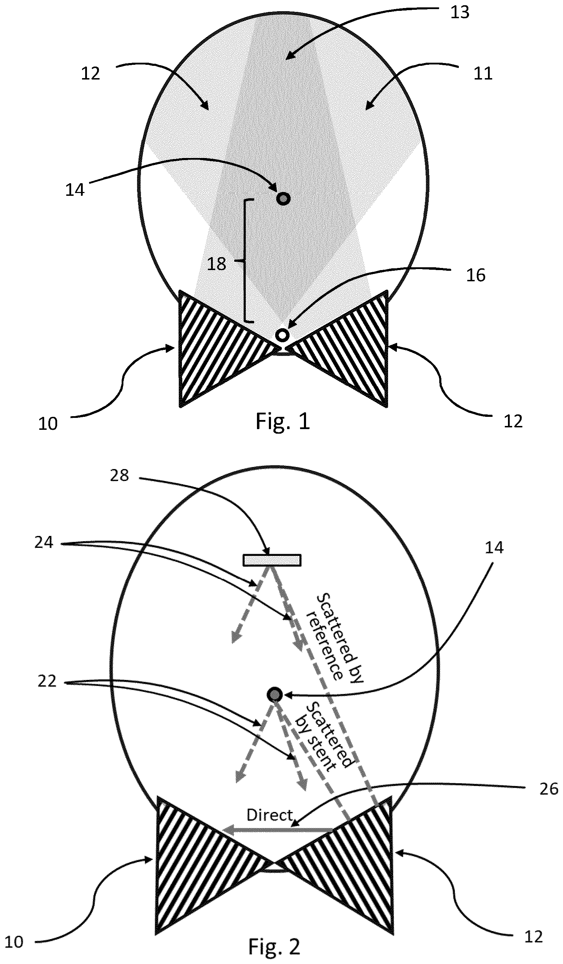

A first aspect of the invention relates to a system to monitor a stent using electromagnetic (EM) radiation, as depicted in Fig 1. In particular, it can detect the integrity of a stent placed in a vessel or body conduct of a patient. By integrity of a stent we refer to the degree at which the mechanical properties of the stent and the tissue surrounding it are maintained with respect to the moment it was placed. Therefore, by keeping its integrity the stent would remain in place, undamaged and unclogged totally or partially, and the same with the tissue surrounding it. Thus, the integrity of a stent would be compromised when said stent suffers any type of structural damage, such as bending, breaking or fracturing, becomes mispositioned, such as travelling inside the duct or vessel of the patient, its lumen is reduced by any means, possibly leading to thrombosis, such as by contracting or accumulating biological matter around or inside it, or if the vessel or ducts in which it is placed suffer any type of in-stent restenosis, including asymmetrical in-stent restenosis, structurally affecting the stent. It is noted that in some embodiments of the invention, the system may merely detect the presence of the stent, or may detect particular conditions such as in-stent restenosis, certain types of stent failures such as fracture, recoil, foreshortening, compression, and others, or any combination thereof.

The system comprises one or more antennas (10 and 12), preferably at least two, which can emit and/or receive electric fields, and a measuring device (30) in electrical communication (302) with the one or more antennas (10 and 12), wherein the measuring device (30) is configured to obtain a frequency response and/or a time response of a first scattered electromagnetic field (24) and/or of a direct electromagnetic field (26). Wherein the first scattered electromagnetic field (24) is preferably launched from at least one emitter antenna (10, 12), scattered by a reference scatterer (28) different than the stent (14), and collected by the same at least one emitter antenna (10, 12) and/or by a different at least one receiver antenna (12, 10) given that the system comprises at least two antennas (10, 12). The direct electromagnetic field (26), on the other hand, is preferably launched from at least one emitter antenna (10, 12), and directly collected by a different at least one receiver antenna (12, 10) given that the system comprises at least two antennas (10, 12).

The measuring device (30) is further configured to obtain a frequency response and/or a time response of a second scattered electromagnetic field (22). Preferably said second scattered electromagnetic field (22) is launched from at least one emitter antenna (10, 12), scattered by the stent (14), and collected by the same at least one emitter antenna (10, 12) or by a different at least one receiver antenna (10, 12), given that the system comprises at least two antennas (10, 12). Wherein in this case emitter and receiver refers to active or passive role of the antenna or antennas in the measurement, which, as mentioned above, in some embodiments can be interchangeable and/or any one antenna can play both roles and emit and receive signal. It is noted that the first and second denotations of the scattered EM field may refer to the scatterer in which the EM is scattered, for example an EM field may be launched by an emitter antenna, and the portion of the EM that is scattered by the stent may be referred to as second scattered EM field (22) and the portion of the EM that is scattered by a reference scatterer may be referred to as first scattered EM field (24). Additionally, it may also refer to different EM fields launched at different times or in different angles or directions, or even the first scattered EM field (24) may refer to a reference measurement or value.

If antennas with sufficient penetration range are used, there is a measurable delay between the direct and scattered propagation paths, or between the propagation paths of the first scattered EM (scattered by a stent) and the second scattered EM (scattered by a reference scatterer), as shown in Fig. 2. This delay depends on the propagation time of the direct and scattered signal, and on the physical mechanisms by which the stent scatters the incident signal which, in turn, depend on the integrity of the stent. This delay can be sensed by transmitting sinusoidal electromagnetic radiation from the emitter antenna and measuring the transmission coefficient to the receiving antenna. Direct and scattered fields gathered at the receiving antenna tend to cancel each other at certain combinations of delays and frequencies of the sinusoidal radiation used. Accordingly, measuring the transmitted EM field and/or transmission coefficient between both antennas at various frequencies captures the delay caused by the stent.

Biological tissues are known to have frequency-dependent electromagnetic properties and, accordingly, scattered fields may depend on the frequency of the emitted signal due to the properties of the tissues surrounding the stent. This dependence may affect both the amplitude and the delay of the scattered signal with respect to the impinging one. Comparing the amplitude and delay of the direct and scattered signals at various frequencies conveys information of the tissues surrounding the stent. From the frequency response and/or the time response of the first scattered electromagnetic field (24) previously obtained, the measuring device can be further configured to determine a representative frequency and an echo delay associated to said first scattered electromagnetic field (24). Similarly, from the frequency response and/or time response of the direct electromagnetic field previously obtained, the measuring device can be configured to determine a representative frequency and an echo delay associated to said direct electromagnetic field (26). Additionally, from the frequency response and/or the time response of the second scattered electromagnetic field (22) previously obtained, the measuring device (30) may be configured to determine a representative frequency and an echo delay associated to said second scattered electromagnetic field (22).

It is noted that the representative frequency may be, for example, a resonance frequency, a frequency value with a local or a global maximum in intensity, an average, or a central point in a fitting function, among other non-limiting options, preferably wherein the representative frequency is the frequency at a global maximum.

Differently to near-field devices, the antennas (10 and 12) of the present invention are co-polarized, therefore they can both receive the electromagnetic radiation emitted by each other. Furthermore, the antennas are suited to monitor distant stents (14) such as coronary stents, which can typically be found at a distance (18) of approximately 3 cm from the body surface, while near-field devices are able only to monitor peripheral stents (16), at a distance under 1 cm.

It is noted that in some embodiments the distance (18) from the stent (14) to the antennas (10 and 12) may be different than the distance (18) shown in Fig. 1, therefore this distance may be longer or shorter, as long as the stent is comprised in the region where both electromagnetic beams (110 and 120) meet (130). The size of the antennas (10 and 12) in Fig. 1 is merely illustrative, as well as the size and the shape of the stent (14), therefore in some other embodiments of the invention they may be bigger or smaller or have different shapes.

It is further noted that the antennas may be manufactured to facilitate equalization. Equalization may be summarized as confronting both antennas (10 and 12) and measuring their frequency response to compensate for their effect when in-vivo stent measurements are performed. Also, in some embodiments, different materials or structures may be placed between the apertures of both antennas (10 and 12) to facilitate equalization.

In order to calculate the integrity of the stent, the measuring device is further configured to compute the parameters mo and ms. It is noted that in some embodiments mo comprises the representative frequency and/or the echo delay associated to the first scattered electromagnetic field (24). It is noted that in some embodiments in addition to the previous embodiment or in combination, $m_D$ comprises the representative frequency and/or the echo delay associated to the direct electromagnetic field (26). Furthermore, the parameter $m_S$ comprises the representative frequency and/or the echo delay associated to the second scattered electromagnetic field (22) component.

It is noted that when indicating that mo and/or mo comprise a representative frequency and/or an echo delay, preferably comprises both, more preferably wherein $m_D$ and/or $m_S$ are directly proportional to both, even more preferably wherein $m_D$ and/or $m_S$ comprise both the representative frequency and the echo delay multiplying each other.

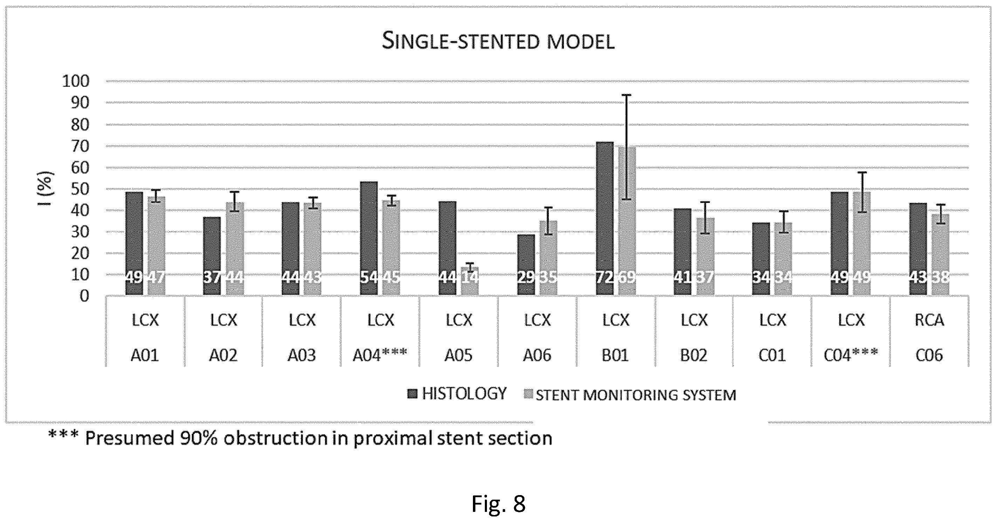

The measuring device (30) and/or the system are preferably configured to compute a mathematical relationship (Is) between the parameter $m_D$ and the parameter $m_S$, preferably wherein Is comprises a ratio between mo and ms. This mathematical relationship $I_S$ is indicative of the integrity of the stent, as backed up by figure 8, wherein the integrity of the stent obtained through this index $I_S$ is compared with histology measurements, that is, the gold standard in the field, and the results obtained are in perfect agreement.

It is noted that the mathematical relationship may comprise other elements, such as numbers or calculated parameters. Preferably the ratio between $m_D$ and $m_S$ is obtained by dividing one by each other, which provides a relationship that allows to readily obtain a percentage.

As is shown in Fig. 2, the antennas (10 and 12) may be configured to receive direct electromagnetic fields (26) from each other and scattered electromagnetic fields from the stent (22) or from a reference scatterer (24). In the example shown in Figure 2, a scattered electromagnetic field (22) is received in a receiver antenna after being launched from an emitter antenna, even though both roles may be exchanged or present at the same time. Similarly, the scattered electromagnetic fields could be launched from an emitter antenna, scattered by the stent and/or a reference scatterer, and received in the same emitter antenna, that also acts as receiver. Again, both antennas may emit and receive the reflected electromagnetic fields due to reciprocity.

Therefore, in an example with two antennas (10 and 12), the electric field transmission coefficient $S_{12}$ may comprise a direct electromagnetic field component launched from a first antenna (10) and collected in a second antenna (12), wherein said electromagnetic field has been conveyed through the body of a patient, and a scattered electromagnetic field component launched from the first antenna (10), scattered by the stent (14) placed inside a vessel or duct of a patient, and collected in the second antenna (12), therefore, it also has been conveyed through the body of the patient. Reciprocally, the electric field transmission coefficient $S_{21}$ comprises a direct electromagnetic field (26) component launched from the second antenna (12) and collected in the first antenna (10) and a scattered electromagnetic field (22) component launched from the second antenna (12), scattered by the stent (14) and collected in the first antenna (10). Preferably, when one of the antennas (10 or 12) acts as emitter, the other one acts as receiver, and vice-versa. More preferably both antennas (10 and 12) act as emitter and receiver at the same time. In other embodiments of the invention only one EM field transmission coefficient may be obtained from one antenna to the other, or instead two transmission coefficients are obtained and an average of both transmission coefficients is performed, any of these may be referred to simply as S. According to a non-limiting example of one embodiment with two antennas (10 and 12), the electric field scattering coefficient $S_{11}$ comprises a scattered electromagnetic field launched from a first antenna (10) and collected in the same first antenna (10), and the electric field scattering coefficient $S_{22}$ comprises a scattered electromagnetic field launched from a second antenna (12) and collected in the same second antenna (12).

It is noted that in some other embodiments of the invention the angle between the direct (26) and scattered (22) or reflected electromagnetic fields emitted and received by the antennas (10 and 12) may be different to the ones in Fig. 2. For instance, the skilled person may adapt it in order to improve the signal received and emitted by the antennas (10 and 12), by increasing or decreasing said angle.

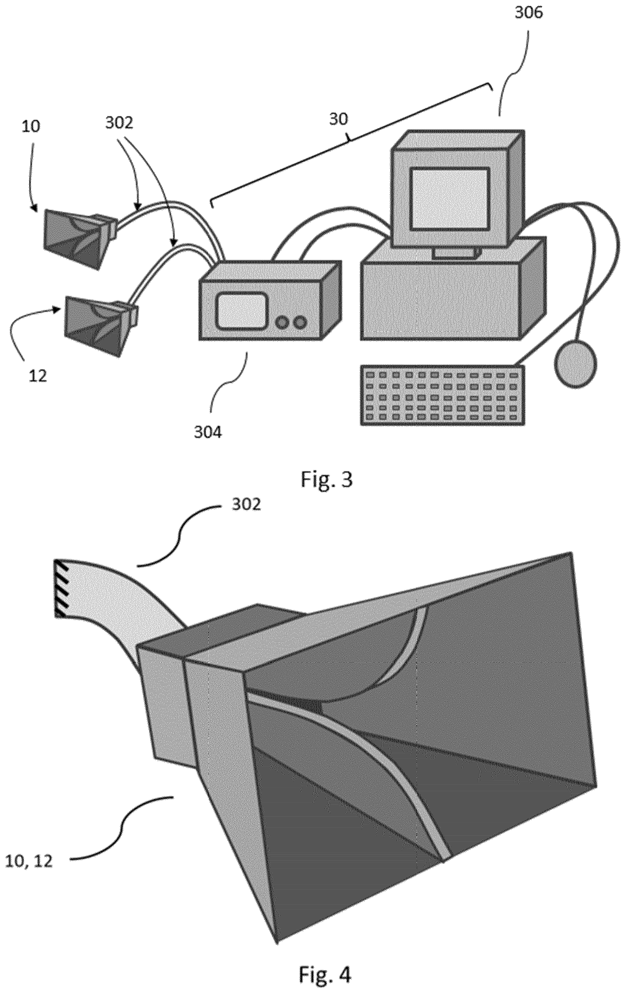

In some embodiments the measuring device (30), as can be seen in Fig. 3, may be configured to determine the electromagnetic field received in one or more antennas (10,12) launched by the same or another one or more emitter antennas (10, 12), said electromagnetic field comprising a direct electromagnetic field (26) component launched from an emitter antenna (10,12) and directly collected by a receiver antenna (10, 12), a scattered electromagnetic field (24) component launched from the emitter antenna (10, 12), scattered by a reference scatterer (28) and collected by the emitter and/or the receiver antenna (12, 10), and/or a scattered electromagnetic field (22) component launched from the emitter antenna (10, 12), scattered by the stent (14) and collected by the emitter and/or the receiver antenna (12, 10). The measuring device (30) may be further configured to obtain a transmission coefficient ($S_{12}$ and/or $S_{21}$ in the two antennas example) between the antennas (10 and 12) by comparing the emitted and received signal, and preferably comprises means for performing a frequency response S(f) of the transmission coefficient $\tau$. It is noted that the role of emitter and receiver antenna may be exchangeable, therefore any of the two or more antennas may emit or receive an electromagnetic signal. The measuring device (30) may also be configured to operate in a one antenna configuration. Wherein the measuring device (30) can determine the electromagnetic field received in one antenna (10, 12) and launched by the same antenna (10, 12), therefore acting as emitter and receiver at the same time, said electromagnetic field comprising a scattered electromagnetic field component launched from an emitter antenna (10, 12), scattered by the stent (14) and collected by the same antenna (10, 12), acting as emitter and receiver at the same time, and comprising a scattered electromagnetic field component launched from an emitter antenna (10, 12), scattered by the a reference scatterer (28) and collected by the same antenna (10, 12), acting as emitter and receiver at the same time. The measuring device (30) may also be configured to operate with two antennas simultaneously in this configuration, and to obtain a scattered coefficient $S_{11}$ and/or $S_{22}$ emitted and received by the same antenna, I, and preferably comprises means for performing a frequency response R(f) of the scattered coefficient $S_{11}$ and/or $S_{22}$.

The measuring device may comprise electronic means (304), computing means (306), or a combination of both. Furthermore, the measuring device may comprise a memory and a processor, embodying instructions stored in the memory and comprising functionality to execute a method of monitoring stents using a system according to any herein disclosed information. For instance, as is shown in fig. 3, the measuring device may comprise a vector network analyser (304) and a computer (306) electrically communicated (302) with the antennas (10, 12). It is noted that the elements shown in Fig. 3 are not proportional for clarity purposes and may or may not be comprised in other embodiments of the invention.

The skilled person may substitute the form in which the electromagnetic fields are transmitted and received from the various antennas. Rather than using electromagnetic fields with sinusoidal time dependence at various frequencies to cover a given frequency range, a broadband time dependence may be used to cover the same frequency range by using deterministic time-domain signals (such as pseudo-random-noise sequences) or random electromagnetic noise. It is further note that in this case, for example, a network analyser may not be employed, instead, some special-purpose transmitters and receivers would preferably be employed.

It is noted that the skilled person may search for the correct placement of the antennas (10, 12) in order to maximize the signal obtained in the measuring device, such as by displacing the antennas (10, 12) along the body of the patient, rotating them with respect to each other to vary the angle at which the electromagnetic fields enter in the body or rotating them both in synchronicity with respect to a fixed point in the plane of contact to align them with the stent.

As it can be appreciated, Fig. 3 shows a system that comprises many other additional characteristics, that may not be comprised in other embodiments or that may be comprised in different ways, for instance the number and type of electronic or computing devices may vary, as well as the number of connections between the different elements of the system, therefore there may be more or less electronic and/or computing devices and more or less connections among them, and thus the skilled person may note that there are multiple variations and alternatives in which a system comprises the features described above.

The measuring device (30) may be further configured to determine, from a frequency response and/or a time response of a direct or scattered electromagnetic field and/or from S(f), a reference frequency $\omega_D$ and an echo time delay, or echo delay, $t_D$, associated to said electromagnetic fields. The skilled person may note that there are different ways in which a measuring device (30) may be configured to determine from the frequency response and/or a time response of a direct or scattered electromagnetic field and/or from S(f) the reference frequency $\omega_D$ and the echo delay $t_D$ associated to the direct and/or scattered electromagnetic fields.

The measuring device (30) therefore may be further configured to compute the parameter $m_D$, and the parameters ms and/or $m_R$, wherein $m_D$ is preferably the reference frequency $\omega_D$ times the echo delay $t_D$ of either or both of the direct electromagnetic field (26) and the first scattered electromagnetic field (24), and $m_S$ is the reference frequency $\omega_S$ times the echo delay $t_S$ of the second scattered electromagnetic fields (22). It is noted that in other embodiments of the invention $m_D$ may comprise only the reference frequency or the echo delay associated to the direct (26) and/or first scattered (24) electromagnetic fields. Correspondingly ms may comprise only the reference frequency associated to the second scattered electromagnetic fields (22), or only the echo delay associated to said EM fields..

Furthermore, in some embodiments the measuring device (30) is configured to compute the relative difference Is between mo and ms wherein Is is indicative of the integrity degree of the stent. For instance, a calibration value of Is taken the moment of the stent implantation in the duct or vessel of a patient, may correlate to an undamaged stent that conserves its integrity. Examples of said possible values could be 0, 0.1, 0.2, etc. (using points as decimal separators), for Is according to one or more embodiments of the invention. It is noted that since Is is a relative difference, it could be expressed as well as a percentage, therefore said possible values could be 0%, 10%, 20%, etc. A stent with a compromised integrity, presenting any damaging condition such as, but not limited to, damage, malposition, thrombosis or ISR, would have a different value of $I_S$ than the one measured when it was just or recently implanted. For example, it could have an decreasing value of Is for different measures taken at different times, indicating a gradual degradation over time. It could also present different increasing and decreasing trends, or any combination thereof, if more than one damaging condition coexist, each condition with a different impact in the value of Is and with different intensities over time. It could also present different slope values, either increasing or decreasing, that could be related to the type of damage that is affecting the integrity of the stent.

Furthermore, the integrity parameter obtained in a measurement, $I_{S-M}$, may be divided or compared with a reference integrity parameter $I_{S-R}$, for example taken at the moment of stent implantation, and a similarity value may be obtained that is also indicative of the integrity of the stent, for instance by finding a ratio between $I_{S-M}$ and

$I_{S-R}$.

It is noted that in some embodiments the measuring device (30) may be comprised or integrated in one of the antennas (10 or 12). Preferably, the antenna (10 or 12) with the integrated measuring device (30) is connected to the other antenna (10 or 12) via a radiofrequency cable. DC cables or wireless connectivity means may connect any of the antennas (10 or 12) and/or the measuring device (30) to a base equipment to provide data transfer.

Advantageously, the system described according to the first aspect of the invention allows the monitorization of the integrity of a stent, comprising information related to stent failures such as, but not restricted to, ISR, fracture, recoil, foreshortening, compression or induced thrombosis in stents placed at depths even longer than 1 cm from the skin, such as coronary stents. Furthermore, it also allows to compare the integrity of a stent over time to check the quality and structural evolution of the stent or similar vascular endoprosthesis.

In a preferred embodiment of the invention, the system comprises at least two antennas in communication with the measuring device (30), wherein the measuring device (30) is configured to:

I. obtain a frequency response and/or a time response, preferably a frequency response, of a direct electromagnetic field (26) launched from at least one emitter antenna (10, 12), and directly collected by a different at least one receiver antenna (12, 10);

II. obtain a frequency response and/or a time response, preferably a frequency response, of a scattered electromagnetic field (22) launched from at least one emitter antenna (10, 12), scattered by the stent (14), and collected by the same emitter antenna (10, 12);

III. determine from the frequency response and/or time response of the direct electromagnetic field (26) obtained in I. a representative frequency and an echo delay associated to said direct electromagnetic field;

IV. determine, from the frequency response and/or the time response of the second scattered electromagnetic field (22) obtained in iii) a representative frequency and an echo delay associated to said second scattered electromagnetic field;

V. compute a mathematical relationship ($m_D$) between the representative frequency and/or the echo delay associated to the direct electromagnetic field (26), preferably by multiplying the representative frequency with the echo delay associated to the direct electromagnetic field (26);

VI. compute a mathematical relationship ($m_S$) between the representative frequency and/or the echo delay associated to the scattered electromagnetic field (22) component, preferably by multiplying the representative frequency with the echo delay associated to the scattered electromagnetic field (22); and

VII. compute a mathematical relationship ($I_s$) between the parameter $m_D$ and the parameter ms, preferably wherein $I_S$ comprises a ratio between mo and ms, wherein the mathematical relationship $I_S$ is indicative of the integrity of the stent.

In a preferred embodiment of the first aspect of the invention, the system comprises two or more antennas (10, 12). In some embodiments an emitter antenna (10, 12) is configured to launch an electromagnetic field (110, 120) onto a stent (14), such that the stent scatters the impinging electromagnetic field (110, 120), and/or such that a guided electromagnetic wave is propagated through at least a portion of the stent. A receiver antenna (12, 10) different than the emitter antenna (10, 12) is configured to collect electromagnetic fields (110, 120), wherein the collected electromagnetic fields comprise direct electromagnetic fields (26) emitted from the emitter antenna and directly collected by the receiver antenna (12, 10) and scattered electromagnetic fields (22) emitted from the emitter antenna (10, 12), scattered from the stent (14) and collected by the receiver antenna (12, 10). The measuring device (30) is in electrical communication (302) with both antennas (10, 12), and is further configured to obtain the frequency response and/or the time response of the collected electromagnetic fields (22, 26) by:

First, measuring an input signal of the emitter antenna (10, 12) and an output signal of the receiver antenna (12, 10), the input signal being related to the electromagnetic field (110, 120) launched onto the stent by the emitter antenna (10, 12) and the output signal being related to the collected electromagnetic fields in the receiver antenna (12, 10).

Secondly, obtaining a scattering coefficient ($S_T$) between the emitter antenna (10, 12) and the receiver antenna (12, 10) by comparing the input signal with the output signal. Therefore, the scattering coefficient ($S_T$) may be a scattering transmission coefficient. The transmission coefficient may be obtained as the ratio between the signal emitted by the emitter antenna and the signal received in the receiver antenna. Due to reciprocity, the scattering coefficient $S_T$ may thus comprise $S_{12}$, $S_{21}$ or any combination thereof, wherein both antennas may act as emitter and/or receiver.

Thirdly performing a frequency response of the scattering coefficient ($S_T$) and/or obtaining a time response S(t) of the scattering coefficient $(S_T)$.. For instance, the frequency response may be obtained by measuring the transmission coefficient as a function of frequency for a given range of frequencies. Preferably, this range includes the average stent resonance frequency (0.7 GHz) in biological tissue, more preferably this range includes the operational frequency range of the antennas [0.5, 3] GHz.

It is noted that both antennas may emit or receive electromagnetic fields, and thus their roles are interchangeable. Furthermore, in some other embodiments of the invention both antennas may act as emitter and receiver, thus obtaining two transmission scattering coefficients $S_{12}$ and $S_{21}$ and thus performing two frequency responses of the scattering coefficients $S_{12}(f)$ and $S_{21}(f)$. An average of $S_{12}(f)$ and $S_{21}(f)$, may be obtained and used as the frequency response S(f). Using the average S(f) presents advantages such as higher precision and lower noise, however any of $S_{12}(f)$, $S_{21}(f)$ and S(f) can be used as frequency response of the scattering transmission coefficient in the system.

Advantageously, this allows to better monitor stents, at depths greater than 1 cm, such as the commercially available ones required in coronary diseases, without needing surgery or additional prosthesis or markers, and to check for the stent integrity in an easy, non-invasive way.

In some embodiments, the measuring device (30) is configured to obtain a frequency response S(f) of at least one scattering coefficient (S) between at least two antennas.

Preferably, the measuring device (30) is further configured to obtain the S-parameters of the scattering matrix of an electromagnetic network. As is depicted in Fig. 1, there may be two antennas (10 and 12), and therefore there may be two transmission coefficients $(S_{12}$ and $S_{21})$ which are the S-parameters corresponding to the ratio between the received EM signal in the first antenna and the emitted EM field by the second antenna, and the ratio between the received EM signal in the second antenna and the emitted EM field by the first antenna, respectively. It is noted that for simplicity, a transmission scattering coefficient will be denominated as $(S_T)$. In other embodiments, there may be more than two antennas (10, 12), and therefore there may be more than two transmission coefficients, one for each combination between two of those antennas. The skilled person will appreciate how to determine those transmission coefficients from the scattering matrix when the system comprises more than two antennas.

The S-parameters of the scattering matrix of an electromagnetic network obtained by the measuring device (30) may comprise also self-coefficients $(S_R)$ corresponding to the ratio between the EM field emitted and received in the same antennas. As is depicted in Fig. 1, there may be two antennas (10 and 12), and therefore there may be two scattering self-coefficients $(S_{11}$ and $S_{22})$ which are the S-parameters corresponding to the ratio between the received EM signal in the first antenna and the emitted EM field by the first antenna, and the ratio between the received EM signal in the second antenna and the emitted EM field by the second antenna, respectively. In a one antenna configuration, it is possible to obtain a scattering coefficient corresponding to the ratio between the received EM and the emitted EM in the same one antenna.

It is noted that in the example with two antennas both antennas may emit or receive electromagnetic fields, and thus their roles are interchangeable. Furthermore, in some other embodiments of the invention both antennas may act as emitter and receiver, thus obtaining two transmission scattering coefficients $S_{12}$ and $S_{21}$ and two scattering coefficients $S_{11}$ and $S_{22}$. Thus, performing two frequency responses of the transmission coefficients $S_{12}(f)$ and $S_{21}(f)$ and two frequency responses of the scattering self-coefficients $S_{11}(f)$ and $S_{22}(f)$. An average of $S_{12}(f)$ and $S_{21}(f)$, and of $S_{11}(f)$ and $S_{22}(f)$, may be obtained and used as the frequency response S(f) or R(f). Using the average frequency response presents advantages such as higher precision and lower noise, however any of $S_{12}(f)$, $S_{21}(f)$ and S(f) can be used as frequency response of the transmission coefficient in the system, and likewise for Sn(f), $S_{22}(f)$ and R(f). In other embodiments with more than two antennas, any combination of emitter and/or receiver antennas may apply as long as it allows to obtain the transmission and reflection coefficients $S_T$ and $S_R$ and their frequency responses.

In a preferred embodiment of the invention, the emitter antenna (10, 12) is configured to collect electromagnetic fields (110, 120) comprising scattered electromagnetic fields (22) from the stent (14), and wherein the measuring device (30) is further configured to obtain the frequency response and/or the time response of the collected electromagnetic fields (110, 120) in the emitter antenna (10, 12) by:

    a. measuring an input signal of the emitter antenna (10, 12) and an output signal of the emitter antenna (10, 12), the input signal being related to the electromagnetic field (110, 120) launched onto the stent by the emitter antenna (10, 12) and the output signal being related to the collected electromagnetic fields in the emitter antenna (12, 10);

    b. obtaining a scattering coefficient $(S_R)$ by comparing the input signal with the output signal of the emitter antenna (10, 12); and

    c. obtaining a frequency response R(f) of the scattering coefficient $(S_R)$ and/or obtaining a time response R(t) of the scattering coefficient $(S_R)$.

According to a preferred embodiment of the first aspect of the invention, the measuring device (30) is configured to determine the reference frequency and the echo delay associated to the direct (26) and scattered (22, 24)) electromagnetic fields. To do so, the measuring device (30) is first configured to obtain the frequency response of the transmission coefficients S(f) in a form for complex numbers.

It is also noted that in some embodiments of the invention, there may be two transmission coefficients related to both antennas acting as emitter and receiver interchangeably, and the frequency response obtained from both may be averaged. Both frequency responses of said transmission parameters $S_{12}(f)$ and $S_{21}(f)$ may be converted to rectangular form before or after averaging them, whereas in other embodiments of the invention only one of them may be used and converted to rectangular form. The frequency response of the transmission parameter is obtained preferably in a frequency span containing the average stent reference frequency (0.7 GHz), more preferably said span ranges between 0.1 and 3 GHz, and even more preferably in a frequency span between 0.01 and 30 GHz.

The measuring device is preferably configured to transform the transmission coefficient S(f) from the frequency domain to the time domain, obtaining a transmission coefficient S(t), using any inverse Fourier transformation, preferably Inverse Fast Fourier Transform (IFFT), to isolate the direct and scattered signals. Finally, the measuring device is configured to fit said time domain transmission coefficient S(t) to the sum of the complex direct and scattered echoes, wherein each of the complex direct and scattered echoes comprise a distribution function times a periodic function. Said direct and scattered components of the transmission coefficient function S(t) are preferably fitted using non-linear curve-fitting in the least-square sense.

It is noted that the skilled person may employ any one of many possible functions to fit the curves, such as, but not limited to, Gaussian, Lorentzian, Cauchy, Poisson, Voigt, Log-Normal, Beta Distribution, Gamma Distribution, Weibull, Student's t-Distribution, Chi-squared, Pearson Type VII, F Distribution or Gumbel Distribution, among many others. It is also noted that the skilled person may employ any of the possible periodic functions to fit the curves, such as, but not limited to, sinusoidal functions, sine and/or cosine functions, square wave, triangle wave, pulse train functions, Jacobi Elliptic functions or Fourier series, among many others.

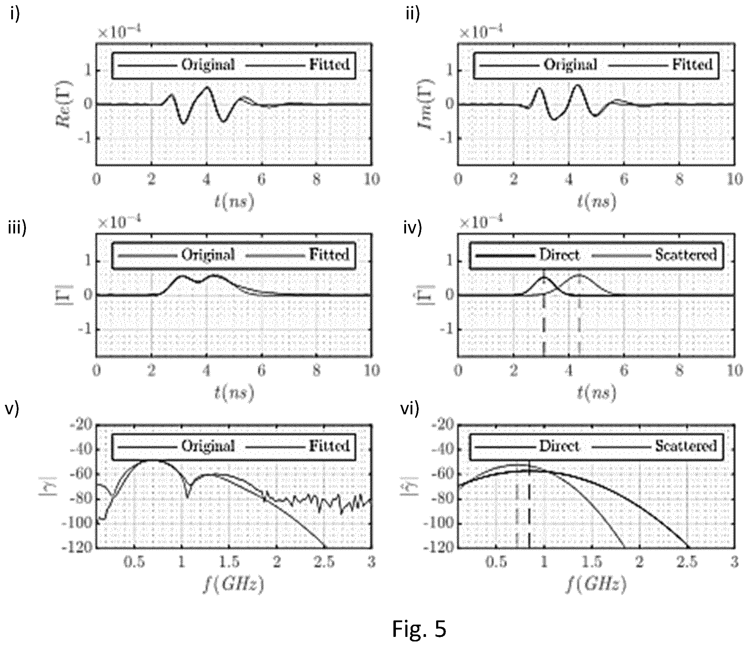

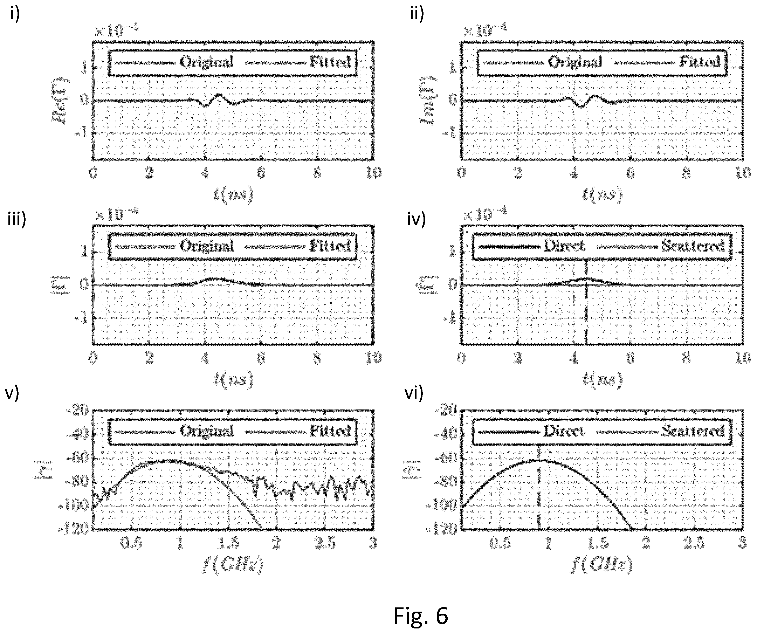

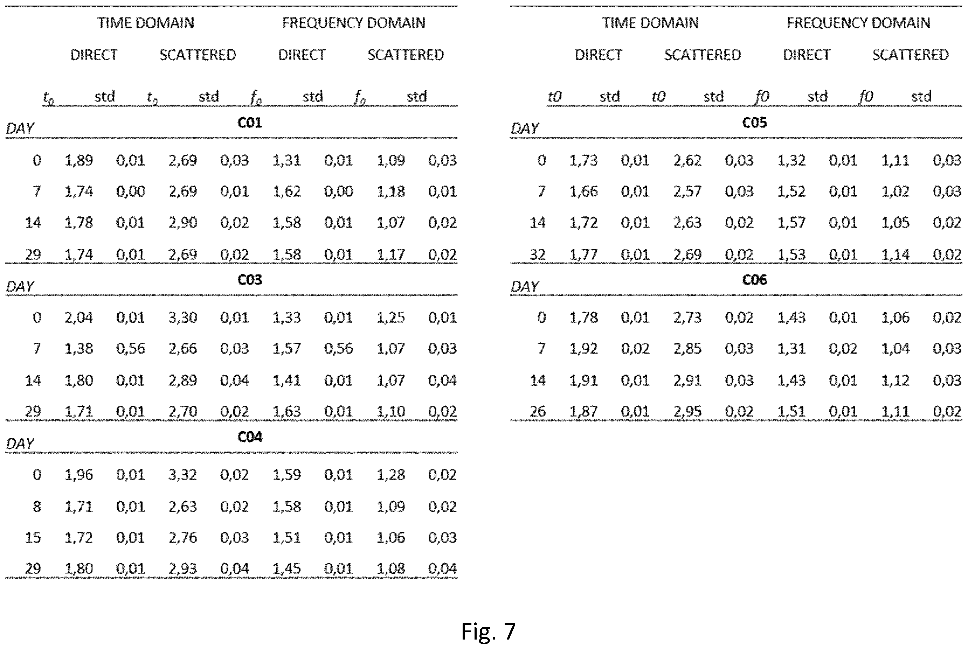

Fig. 5 (v) shows a measured (original) frequency response of the transmission coefficient S(f) in the presence of a stent and a corresponding function fitting. It is noted that the presence of the stent can be detected by the notch at around 1.1 GHz, and figure 5 (iii) shows the echo delay of the scattered electromagnetic field around 4.3 ns. On the other hand, Fig. 6 (v) shows a measurement of the frequency response of the transmission coefficient S(f) without a stent, where no notch and only one local maximum can be appreciated, indicating that there is no stent prosthesis. Fig. 5 (i) shows the fitting and the original time domain transmission coefficient S(t) in the presence of a stent, whereas Fig. 6 (i) shows the fitting and the original time domain transmission coefficient S(t) without a stent. Noteworthy, the amplitude is bigger in the presence of stent in Fig. 5 (i) due to the addition of the direct and scattered signal, while in Fig. 6 (i) there is only the direct component, since there is no stent to scatter the electromagnetic fields. Figure 7 summarizes the obtained values of echo times and reference frequencies and their standard deviation for different days since the placement of the coronary stent (0, 3, 7, 14, 20 and 34 days) in 6 pigs (P03, P04, P05, P06, P07 and P09).

The stent presence can also be detected by comparing the difference in values for a basal measurement, i.e., without stent, , with a measurement in the presence of stent.

It is noted that arithmetically combining these echo delay and reference frequency of the scattered signal and those of the direct signal as a normalization factor allows computing a descriptive index, named $I_S$, that strongly relates to the stent status. The $I_S$ index allows to quantify stent-related pathologies like, but not limited to, the amount of restenotic or thrombothic tissue present in the stent at the time of measurement or abruptly changing in the event of an architectural stent fault.

Advantageously, fitting the real and imaginary parts of the transmission coefficient in the frequency domain S(f) and in the time domain S(t) allows to readily obtain the reference frequencies and echo times for the direct (26) and scattered (22) electromagnetic fields. Furthermore, finding these parameters allows to detect the presence or not of a stent (14) due to the difference in average results between direct electromagnetic field parameters. Also, it is possible to determine the presence of a stent (14) based on the identification or not of a pronounced notch (Fig. 5 (v)) in the frequency response of the transmission coefficient.

In a preferred embodiment of the invention, obtaining the frequency response of a collected electromagnetic field comprises launching a fixed-frequency electromagnetic field (110, 120) from the emitter antenna (10, 12) for a period of time T and for two or more frequencies comprised in a range of frequencies R, or launching a frequency modulated electromagnetic field (110, 120) from the emitter antenna (10, 12) for a period of time T' and wherein the frequency-modulated electromagnetic field (110, 120) continuously varies over the frequencies comprised in a frequency range R', or launching an electromagnetic field having a spreading modulation (110, 120) from the emitter antenna (10, 12) for a period of time T", wherein the spreading-modulated electromagnetic field (110, 120) covers a frequency range R"; and measuring the collected electromagnetic fields in the emitter and/or in the receiver antenna (10, 12).

According to another preferred embodiment of the invention, the measuring device (30) is configured to determine, from S(t), the reference frequency and the echo delay associated to the direct (26) and scattered (22) electromagnetic fields by a) directly obtaining the time domain response of the transmission coefficient S(t), preferably through ultra-wideband (UWB) pulses, and b) fitting the time domain transmission coefficient S(t) obtained in step (a) to the sum of the complex direct and scattered echoes, wherein each of the complex direct and scattered echoes comprise a distribution function times a periodic function. Therefore, in some embodiments performing the time response of a collected electromagnetic field comprises launching an ultra wide-band (UWB) electromagnetic impulse from the emitter antenna (10, 12) for a period of time T"", wherein the UWB impulse comprises the frequencies comprised in the frequency range R"", and measuring the collected electromagnetic fields in the emitter (10, 12) and/or the receiver antenna (12, 10).

Advantageously, this configuration of the measuring device (30) allows to simplify the steps and employ other measuring techniques to directly obtain the time domain response of the transmission coefficient S(t) from a time domain measurement, skipping the step of measuring a frequency domain transmission coefficient S(f) and converting it to the time domain through an inverse Fourier transformation, such as, for instance, through Inverse Fast Fourier Transform.

According to a more preferred embodiment, the system may be configured to average the frequency response of the transmission coefficients measured in at least two antennas (10, 12). It is noted that said average may be obtained before or after converting the signal to the time domain using an inverse Fourier transformation, preferably Inverse Fast Fourier Transformation (IFFT). Averaging the signal is beneficial because it reduces noise and improves precision, however in other embodiments of the invention said frequency response of the transmission coefficient may not be averaged, and instead only one of them may be measured or chosen. Preferably, the frequencies are averaged according to the corresponding relative amplitude between the emitted and received electromagnetic fields (22, 24, 26).

In a preferred embodiment, the scattering coefficient ($S_T$) is obtained as a complex ratio between the electromagnetic field launched from the emitter antenna (10, 12) and the electromagnetic field collected in the receiver antenna (12, 10), and the scattering coefficient ($S_R$) is obtained as a complex ratio between the electromagnetic field launched from the emitter antenna (10, 12) and the electromagnetic field collected in the same emitter antenna (10, 12

Optionally, the magnitude of the time domain transmission coefficient |S(t)|, Fig. 5 (iii), may be fitted to a two term Gaussian function as displayed in Fig. 5 (iv), corresponding each to the direct and scattered echoes' magnitudes respectively, to estimate one or more of the parameters that define the gaussian function. Said estimated parameters can serve as starting point for the fitting of the time domain transmission coefficient S(t).

Optionally, the real and the imaginary parts of the time domain transmission coefficient S(t), may be windowed by the direct echo magnitude, and fitted to a sinusoidal function each, to estimate one or more of the parameters $\omega_D$ and $\gamma_D$. Said estimated parameters may serve as starting point for the fitting of the time domain transmission coefficient S(t).

Optionally, the real and the imaginary parts of the time domain transmission coefficient S(t), may be windowed by the scattered echo magnitude, and fitted to a sinusoidal function each, to estimate one or more of the parameters $\omega_S$ and $\gamma_S$. Said estimated parameters may serve as starting point for the fitting of the time domain transmission coefficient S(t).

Optionally, the fitting of the parameters in the time domain transmission coefficient S(t) may be constrained with respect to the estimated parameters obtained through the fitting to a two term Gaussian function.

Advantageously, constraining the fitting of the parameters allows the final fit to be realistic and not diverge too much from the estimated parameters.

In another preferred embodiment of the invention, the relative difference (Is) is computed as:

$$I_S(\%) = \left(1 - \frac{m_D}{m_S}\right)$$

Fig. 8, shows the integrity percentage of a stent placed in several experimental pigs (A01, A02, A03, A04, A05, A06, B01, B02, C01, C04 and C06) measured according to histology and according to the method and system of the invention [please, provide more details regarding the specimens, times and conditions in figure 8]. As can be seen in Fig. 8, the comparison of the observed integrity degree of a stent, , obtained from the relative difference $I_S$ computed as the formula above, and from histological data, shows a strong correlation among most of the experimental cases except for A05, for which less measurements were obtained. This supports the validity of the parameter $I_S$ (%) as an estimator of the integrity degree of a stent, which can be correlated to the ISR degree, as is the case in the particular example of figure 8.

In some other embodiments of the invention, the relative difference $I_S$ between $m_D$ and $m_S$ may be indicative of structural damage on the stent, such as, but not restricted to, fracture, recoil, foreshortening or compression.

Advantageously, this parameter allows a precise and easy way to obtain the estimation of the integrity degree of a stent which is related to conditions such as, but not limited to, ISR, stent degradation, malposition, among many others, and allows to monitor the evolution of said stent by a non-invasive and ready to use system.

According to a preferred embodiment of the invention, all the frequency and/or time response measurements are normalized by being divided by an equalizing transmission scattering coefficient. Preferably said equalizing transmission scattering coefficient is a reference measurement and/or a measurement between two antennas (10, 12) when said two antennas are facing each other. More preferably the reference measurement is a measurement from one or more antennas in a reference medium, from a basal-like structure or from a structure with pre-defined electromagnetic properties. According to another preferred embodiment of the invention, the antennas (10, 12) are co-polarised. It is noted that the antennas may be polarised linearly, circularly, elliptically, or in any other configuration as long as it allows both antennas to receive the signal emitted by the reciprocal antenna (10, 12). Preferably, the antennas (10, 12) are linearly polarised, more preferably at a frequency span that includes the average stent resonance frequency in biological tissue (0.7 GHz), preferably the span ranges between 0.1 and 3 GHz. Advantageously, this allows to receive the electromagnetic waves conveyed directly from the emitter antenna (10, 12), contrary to what happens in near-field probes, where the transmission coefficient is null without a stent in its surroundings. Furthermore, by separating the super position of the direct and scattered waves, it is possible to monitor the integrity. In another preferred embodiment of the invention, the integrity degree of the stent obtained by the system according to the first aspect of the invention is scaled by a factor X, wherein the scaling factor X depends on the physical antennas (10, 12), the dielectric biomimetic filling and/or the calibration of the system. For instance, in Fig. 8 a scaling factor was used of X = 2.00. Differences between the system employed in both measurements are the initial calibration of the measuring device and the dielectric biomimetic filling of the antennas. It is noted that the skilled person may identify a different scaling factor X according to their particular implementation of the system such as calibration conditions, type of filling for the antennas or degradation level of said filling, or the type of antennas employed, as well as ambient conditions such as humidity or temperature.

Advantageously, this allows for a correct estimation of the parameter $I_S$ or $I_R$, indicative of the integrity degree of a stent, specially adapted to the particular implementation of the system which involves aspects such as the type of antennas (10, 12) used, the dielectric biomimetic filling chosen and the calibration conditions.

According to another preferred embodiment, the measuring device (30) is configured to measure the input and output signals in synchrony with the heartbeat of the subject comprising the stent. This allows to reproduce the same conditions at every measurement point, as the blood pressure of the vessel where the stent is placed changes at the different stages of the heartbeat cycle, concomitantly changing the volume of blood, the dielectric constant, the propagation path of the electromagnetic fields, and the concomitant stent deformation, affecting thus the measured transmission coefficient unless the emitting signals are synchronized with the heartbeat. In some embodiments of the invention an ECG signal may be directly used as a trigger signal, whereas in other embodiments a conveniently transformed ECG signal may be used as a trigger signal, wherein this trigger signal is provided to the measuring device to launch the electromagnetic fields. The transformation of the ECG signal to a trigger signal may be performed with custom scripts or by commercially available devices.

Advantageously, synchronizing the measurement of the transmission coefficient with the heartbeat allows to remove measurement artifacts coming from the different blood pressures in the body vessels at the different stages of the heartbeat, such as, for instance, in coronary arteries, making the measurements reliable and reproducible.

In a more preferred embodiment, the system further comprises means for obtaining the ECG of the subject, and the measuring device (30) is configured to receive the ECG signal from the means for obtaining the ECG. It is noted that the measuring device (30) may be configured to treat, modify or transform the signal directly received by the means for obtaining an ECG in such a way that the resulting signal can trigger the transmission coefficient measurements and the emission and reception of the electromagnetic fields.

Advantageously, this way the system is able to monitor the heartbeat, and the measuring device (30) is able to trigger the signals in synchrony with said heartbeat. In another preferred embodiment of the invention, the antennas (10, 12) are extended Gap Ridge Horn (EGRH) antennas (10, 12), as shown in Fig. 4. It is noted that the EGRH (10, 12) shown in the picture may comprise metal in its composition, and they may adopt different configurations, for instance, in some embodiments, the lateral walls may be grids or bars, or present curved or smooth shapes, or instead of pyramidal they present circular, rectangular, or prismatic shapes, among others. Also, the proportions displayed in Fig. 4 are just for clarity purposes, and the walls, as well as the ridges may comprise different lengths and thicknesses, and may be made of different materials with respect to each other.

Advantageously, these antennas (10, 12) allow for an improved directivity of the signal and thus the possibility to direct the electromagnetic fields into the stent (14), provide a broad bandwidth that covers the reference frequencies of the stent, and the reference frequencies of the direct and scattered electromagnetic fields in biological tissue, as well as require a simple construction and adjustment.

In another embodiment, the antennas (10, 12) comprise a relative angle with respect to each other such that their focal electromagnetic field overlaps further than 1 cm from the probe itself. It is noted that the relative angle respective to each other may vary depending on the distance between antennas (10, 12) and the depth of the stent. It is understood that a skilled person may try different positions until finding the correct angle that yields the maximum signal.

Advantageously, this allows the monitoring of stents (14) for distances above 1 cm, such as coronary stents which are placed at around 3 cm.

According to another embodiment, the antennas (10, 12) are linearly polarised at a frequency span that includes the average stent reference frequency in biological tissue (0.7 GHz). Preferably the frequency span ranges between 0.01 and 30 GHz, more preferably between 0.1 and 3 GHz, and it is noted that many other ranges may be valid as long as they include the stent reference frequency in biological tissue.

Advantageously, this allows the receiver antenna (10, 12) to receive direct electromagnetic radiation (26) emitted from the emitter antenna and scattered electromagnetic radiation (22) from the stent (14), since the co-polarized antennas can receive direct electromagnetic radiation from other antennas and the range of polarization includes the reference frequency of the stent (14), allowing thus the stent (14) to scatter the signal strongly enough to be received by the receiver antenna (10, 12).

According to a preferred embodiment of the first aspect of the invention, $m_D$ comprises or consists of the representative frequency times the echo delay of the direct electromagnetic field (26), launched from an emitter antenna (10, 12) and directly collected by a receiver antenna (12, 10) different than the emitter antenna (10, 12), and ms comprises or consists of the representative frequency times the echo delay of the scattered electromagnetic field (22) component launched from an emitter antenna (10, 12), scattered by the stent (14) and collected by a receiver antenna (12, 10) different than the emitter antenna (10, 12), optionally wherein the parameter $m_S$ further comprises or is averaged with the representative frequency of the electromagnetic field launched from an emitter antenna (10, 12), reflected and/or scattered by the stent (14) and collected by the same antenna (10, 12).

Preferably wherein $m_S$ comprises a representative frequency and/or an echo delay associated to a simulation or in vitro measurements of different stent and/or lesion models, in addition to, or instead of the second scattered electromagnetic field (22) component.

A second aspect of the invention relates to a method to monitor stents (14) according to any of the systems of the first aspect of the invention, for example, the system comprising one or more antennas (10, 12) and a measuring device (30) in communication (302) with the one or more antennas (10, 12). The method comprises the following steps:

    i. obtaining a frequency response and/or a time response of a first scattered electromagnetic field (24) launched from at least one emitter antenna (10, 12), scattered by a reference scatterer (28) different than the stent (14), and collected by the same at least one emitter antenna (10, 12) and/or in case that the system comprises at least two antennas (10, 12), collected by a different at least one receiver antenna (12, 10) ; and/or

    ii. obtaining a frequency response and/or a time response of a direct electromagnetic field (26) launched from at least one emitter antenna (10, 12), and directly collected by a different at least one receiver antenna (12, 10) in case that the system comprises at least two antennas (10, 12);

    iii. obtaining a frequency response and/or a time response of a second scattered electromagnetic field (22) launched from at least one emitter antenna (10, 12), scattered by the stent (14), and collected by the same emitter antenna (10, 12) or by a different at least one receiver antenna (10, 12) in case that the system comprises at least two antennas (10, 12);

    iv. determining, from the frequency response and/or the time response of the first scattered electromagnetic field (24) obtained in i), a representative frequency and an echo delay associated to said first scattered electromagnetic field (24), and/or determine from the frequency response and/or time response of the direct electromagnetic field (26) obtained in ii), a representative frequency and an echo delay associated to said direct electromagnetic field;

    v. determining, from the frequency response and/or the time response of the second scattered electromagnetic field (22) obtained in iii) a representative frequency and an echo delay associated to said second scattered electromagnetic field;

    vi. computing a mathematical relationship ($m_D$) between the representative frequency and/or the echo delay associated to the first scattered electromagnetic field (24), and/or between the representative frequency and/or the echo delay associated to the direct electromagnetic field (26);

    vii. computing a mathematical relationship (ms) between the representative frequency and/or the echo delay associated to the second scattered electromagnetic field (22) component; and

    viii. computing a mathematical relationship ($I_S$) between the parameter $m_D$ and the parameter ms, preferably wherein $I_S$ comprises a ratio between $m_D$ and ms,

wherein the mathematical relationship $I_S$ is indicative of the integrity of the stent. The mathematical relationship Is can therefore be compared with reference values (from previous measurements of said stent or a different stent, tabulated values and/or mathematically modelled values, among other options) to monitor the integrity of the stent.

In a preferred embodiment of the method of the second aspect of the invention, for example wherein the system comprises two or more antennas, wherein an emitter antenna (10, 12) is configured to launch an electromagnetic field (110, 120) onto a stent (14), such that the stent scatters the impinging electromagnetic field (110, 120) and wherein a receiver antenna (12, 10) different than the emitter antenna (10, 12) is configured to collect electromagnetic fields (110, 120) that comprise direct electromagnetic fields (26) emitted from the emitter antenna and directly collected by the receiver antenna (12, 10) and scattered electromagnetic fields (22) emitted from the emitter antenna (10, 12), scattered from the stent (14) and collected by the receiver antenna (12, 10), the method further comprises the steps of:

    a. measuring an input signal of the emitter antenna (10, 12) and measuring an output signal of the receiver antenna (12, 10), the input signal being related to the electromagnetic field (110, 120) launched onto the stent by the emitter antenna (10, 12) and the output signal being related to the collected electromagnetic fields in the receiver antenna (12, 10);

    b. obtaining a scattering coefficient ($S_T$) between the emitter antenna (10, 12) and the receiver antenna (12, 10) by comparing the input signal with the output signal; and

    c. obtaining a frequency response S(f) of the scattering coefficient ($S_T$) and/or obtaining a time response S(t) of the scattering coefficient ($S_T$).

In a more preferred embodiment of the method of the second aspect of the invention, for example wherein the emitter antenna (10, 12) is configured to collect electromagnetic fields (110, 120) comprising scattered electromagnetic fields (22) from the stent (14), the method further comprises the steps of:

    d. measuring an input signal of the emitter antenna (10, 12) and an output signal of the emitter antenna (10, 12), the input signal being related to the electromagnetic field (110, 120) launched onto the stent by the emitter antenna (10, 12) and the output signal being related to the collected electromagnetic fields in the emitter antenna (12, 10);

    e. obtaining a scattering coefficient ($S_R$) by comparing the input signal with the output signal of the emitter antenna (10, 12); and

    f. obtaining a frequency response R(f) of the scattering coefficient ($S_R$) and/or obtaining a time response R(t) of the scattering coefficient ($S_R$).

According to a preferred embodiment of the method of the second aspect of the invention, wherein the system comprises at least two antennas in communication with the measuring device (30), the method comprises the steps of:

    (I) obtaining a frequency response and/or a time response of a direct electromagnetic field (26) launched from at least one emitter antenna (10, 12), and directly collected by a different at least one receiver antenna (12, 10);

    (II) obtaining a frequency response and/or a time response of a scattered electromagnetic field (22) launched from at least one emitter antenna (10, 12), scattered by the stent (14), and collected by the same emitter antenna (10, 12);

    (III) determining from the frequency response and/or time response of the direct electromagnetic field (26) obtained in I. a representative frequency and an echo delay associated to said direct electromagnetic field;

    (IV) determining, from the frequency response and/or the time response of the second scattered electromagnetic field (22) obtained in iii) a representative frequency and an echo delay associated to said second scattered electromagnetic field;

    (V) computing a mathematical relationship ($m_D$) between the representative frequency and/or the echo delay associated to the direct electromagnetic field (26);

    (VI) computing a mathematical relationship (ms) between the representative frequency and/or the echo delay associated to the scattered electromagnetic field (22) component; and

    (VII) computing a mathematical relationship ($I_S$) between the parameter $m_D$ and the parameter ms, preferably wherein $I_S$ comprises a ratio between $m_D$ and ms, wherein the mathematical relationship $I_S$ is indicative of the integrity of the stent.

The mathematical relationship $I_S$ may therefore be compared with reference values (from previous measurements of said stent or a different stent, tabulated values and/or mathematically modelled values, among other options) to monitor the integrity of the stent.

All of the above are fully within the scope of the present disclosure and are considered to form the basis for alternative embodiments in which one or more combinations of the above-described features are applied, without limitation to the specific combination disclosed above.

In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. All such equivalents, modifications or adaptations fall within the scope of the present disclosure.

**Examples**

Materials and methods

To acquire a measurement, EGRHs are connected via coaxial cables to a two-port Vector Network Analyzer (VNA) that triggers the acquisition of every single point of the trace (101 points) with every heartbeat. Thus, every point is acquired at the same phase of the cardiac cycle, ensuring stability in the deformation of the stent caused by the heart motion. For such synchronization to be possible, a custom-made script transforms the Electrocardiogram (ECG) signal from the multiparametric external output into a TTL signal send to the trigger input of the VNA. Additionally, to aid during the process, the VNA is controlled by a computer that can setup the instrument, launch a measurement and save the traces locally in a custom format. The computer is also connected to a digital oscilloscope that plots both the ECG and the TTL signals to validate its synchronicity. A measurement is comprised by the magnitude and phase of the four S-parameters ($s_{11}$, $s_{12}$, $s_{21}$, and $s_{22}$) in the frequency span $f$ = [0.13] GHz of interest. Results of the measurements as a function of frequency and time can be observed in figure 5 for a specimen with an stent implanted, and in figure 6 for a control specimen without stent.

Right before starting an experiment, antennas are filled with a home-made material (phantom) that emulates the dielectric properties (electrical permittivity and conductivity) of biological tissues. Phantom is enclosed in the EGRH's cavity by an adhesive sheet and left there during the whole procedure (35 days).

The pre-clinical experiments described in figures 5 to 8 were performed as aforementioned by connecting every independent instrument.

Aiming to monitor stent prosthetic devices, and in-stent restenosis (ISR), the EGRH antennas were used to perform two *in vivo* experiments. Six (C01, C03, C04, C05 and C06), six (A01, A02, A03, A04, A05 and A06) and two (B01 and B02) crossbreed Landrace X Large White pigs were measured during one month. All of the animals underwent an angioplasty procedure with stent implantation and one animal was left without stent (control).

During the first trial, the animals were kept alive for 35 days and measured on day 0 (stent implantation), day 7, 14, 21 (follow-up) and day 29 (in some cases day 26 or say 32), the sacrifice, as can be seen in figure 7. On day 0 measurements were acquired before (basal) and after the implantation procedure. Measurements were performed in an operating room with the anesthetized animal in supine position. For every specimen and on both right and left sides of the animal, EGRH antennas were placed over the torso. Measurements taken at the right side of the animal simulated a basal acquisition after the stent implantation. In addition, to track the phantom degradation along the experiment, a control measurement was performed each day facing the two horn apertures of the phantom-filled antennas. In figure 8 it can be observed the stent integrity measured on the sacrificial day with the system and method of the invention (stent monitoring system) and with histology.

**Claims**

1. A system configured to monitor the integrity of a stent (14), the system comprising one or more antennas (10, 12) and a measuring device (30) in communication (302) with the one or more antennas (10, 12), wherein the measuring device (30) is configured to:

   i. obtain a frequency response and/or a time response of a first scattered electromagnetic field (24) launched from at least one emitter antenna (10, 12), scattered by a reference scatterer (28) different than the stent (14), and collected by the same at least one emitter antenna (10, 12) and/or in case that the system comprises at least two antennas (10, 12), collected by a different at least one receiver antenna (12, 10) ; and/or
   ii. obtain a frequency response and/or a time response of a direct electromagnetic field (26) launched from at least one emitter antenna (10, 12), and directly collected by a different at least one receiver antenna (12, 10) in case that the system comprises at least two antennas (10, 12);

   wherein the measuring device (30) is further configured to:

   iii. obtain a frequency response and/or a time response of a second scattered electromagnetic field (22) launched from at least one emitter antenna (10, 12), scattered by the stent (14), and collected by the same emitter antenna (10, 12) or by a different at least one receiver antenna (10, 12) in case that the system comprises at least two antennas (10, 12);
   iv. determine, from the frequency response and/or the time response of the first scattered electromagnetic field (24) obtained in i), a representative frequency and an echo delay associated to said first scattered electromagnetic field (24), and/or determine from the frequency response and/or time response of the direct electromagnetic field (26) obtained in ii), a representative frequency and an echo delay associated to said direct electromagnetic field;

v. determine, from the frequency response and/or the time response of the second scattered electromagnetic field (22) obtained in iii) a representative frequency and an echo delay associated to said second scattered electromagnetic field;

vi. compute a mathematical relationship ($m_D$) between the representative frequency and/or the echo delay associated to the first scattered electromagnetic field (24), and/or between the representative frequency and/or the echo delay associated to the direct electromagnetic field (26);

vii. compute a mathematical relationship (ms) between the representative frequency and/or the echo delay associated to the second scattered electromagnetic field (22) component; and

viii. compute a mathematical relationship ($I_S$) between the parameter $m_D$ and the parameter ms, preferably wherein $I_S$ comprises a ratio between $m_D$ and ms,

wherein the mathematical relationship $I_S$ is indicative of the integrity of the stent.

2. The system according to claim 1 in the case that the system comprises two or more antennas, wherein an emitter antenna (10, 12) is configured to launch an electromagnetic field (110, 120) onto a stent (14), such that the stent scatters the impinging electromagnetic field (110, 120),

wherein a receiver antenna (12, 10) different than the emitter antenna (10, 12) is configured to collect electromagnetic fields (110, 120), wherein the collected electromagnetic fields comprise direct electromagnetic fields (26) emitted from the emitter antenna and directly collected by the receiver antenna (12, 10) and scattered electromagnetic fields (22) emitted from the emitter antenna (10, 12), scattered from the stent (14) and collected by the receiver antenna (12, 10),

and wherein the measuring device (30) is further configured to obtain the frequency response and/or the time response of the collected electromagnetic fields (110, 120) in the receiver antenna (12, 10) by:

a. measuring an input signal of the emitter antenna (10, 12) and measuring an output signal of the receiver antenna (12, 10), the input signal being related to the electromagnetic field (110, 120) launched onto the stent by the emitter antenna (10, 12) and the output signal being related to the collected electromagnetic fields in the receiver antenna (12, 10);

b. obtaining a scattering coefficient ($S_T$) between the emitter antenna (10, 12) and the receiver antenna (12, 10) by comparing the input signal with the output signal; and

c. obtaining a frequency response S(f) of the scattering coefficient ($S_T$) and/or obtaining a time response S(t) of the scattering coefficient ($S_T$).

3. The system according to claim 2, wherein the emitter antenna (10, 12) is configured to collect electromagnetic fields (110, 120) comprising scattered electromagnetic fields (22) from the stent (14),

and wherein the measuring device (30) is further configured to obtain the frequency response and/or the time response of the collected electromagnetic fields (110, 120) in the emitter antenna (10, 12) by:

d. measuring an input signal of the emitter antenna (10, 12) and an output signal of the emitter antenna (10, 12), the input signal being related to the electromagnetic field (110, 120) launched onto the stent by the emitter antenna (10, 12) and the output signal being related to the collected electromagnetic fields in the emitter antenna (12, 10);

e. obtaining a scattering coefficient ($S_R$) by comparing the input signal with the output signal of the emitter antenna (10, 12); and

f. obtaining a frequency response R(f) of the scattering coefficient ($S_R$) and/or obtaining a time response R(t) of the scattering coefficient ($S_R$).

4. The system according to claim 1, wherein the system comprises at least two antennas in communication with the measuring device (30), wherein the measuring device (30) is configured to:

(I) obtain a frequency response and/or a time response of a direct electromagnetic field (26) launched from at least one emitter antenna (10, 12), and directly collected by a different at least one receiver antenna (12, 10);

(II) obtain a frequency response and/or a time response of a scattered electromagnetic field (22) launched from at least one emitter antenna (10, 12), scattered by the stent (14), and collected by the same emitter antenna (10, 12);

(III) determine from the frequency response and/or time response of the direct electromagnetic field (26) obtained in I. a representative frequency and an echo delay associated to said direct electromagnetic field;

(IV) determine, from the frequency response and/or the time response of the second scattered electromagnetic field (22) obtained in iii) a representative frequency and an echo delay associated to said second scattered electromagnetic field;

(V) compute a mathematical relationship ($m_D$) between the representative frequency and/or the echo delay associated to the direct electromagnetic field (26);

(VI) compute a mathematical relationship (ms) between the representative frequency and/or the echo delay associated to the scattered electromagnetic field (22) component; and

(VII) compute a mathematical relationship ($I_S$) between the parameter $m_D$ and the parameter ms, preferably wherein $I_S$ comprises a ratio between $m_D$ and $m_S$, wherein the mathematical relationship $I_S$ is indicative of the integrity of the stent.

5. The system according to any one of the previous claims, wherein obtaining the frequency response of a collected electromagnetic field comprises launching a fixed-frequency electromagnetic field (110, 120) from the emitter antenna (10, 12) for a period of time T and for two or more frequencies comprised in a range of frequencies R, or launching a frequency modulated electromagnetic field (110, 120) from the emitter antenna (10, 12) for a period of time T' and wherein the frequency-modulated electromagnetic field (110, 120) continuously varies over the frequencies comprised in a frequency range R', or launching an electromagnetic field having a spreading modulation (110, 120) from the emitter antenna (10, 12) for a period of time T", wherein the spreading-modulated electromagnetic field (110, 120) covers a frequency range R"; and measuring the collected electromagnetic fields in the emitter and/or in the receiver antenna (10, 12).

6. The system according to any one of the previous claims, wherein performing the time response of a collected electromagnetic field comprises launching an ultra wide-band (UWB) electromagnetic impulse from the emitter antenna (10, 12) for a period of time T"", wherein the UWB impulse comprises the frequencies comprised in the frequency range R"", and measuring the collected electromagnetic fields in the emitter (10, 12) and/or the receiver antenna (12, 10).

7. The system according to any one of the previous claims, wherein the representative frequencies associated to the direct (26) and first and second scattered (22, 24) electromagnetic fields are the weighted relative average of the frequencies of said electromagnetic fields (22, 24, 26), wherein the frequencies are averaged according to the corresponding relative amplitude between the emitted and received electromagnetic fields (22, 24, 26).

8. The system according to any of claims 2 to 6, wherein the scattering coefficient ($S_T$) is obtained as a complex ratio between the electromagnetic field launched from the emitter antenna (10, 12) and the electromagnetic field collected in the receiver antenna (12, 10), and the scattering coefficient ($S_R$) is obtained as a complex ratio between the electromagnetic field launched from the emitter antenna (10, 12) and the electromagnetic field collected in the same emitter antenna (10, 12).

9. The system according to any of the previous claims, wherein the mathematical relationship ($I_S$) is computed as or proportional to

$$I_S(\%) = \left(1 - \frac{m_D}{m_S}\right)$$

10. The system according to any of the previous claims wherein all the frequency and/or time response measurements are normalized by being divided by an equalizing scattering coefficient.

11. The system according to any of the previous claims, wherein the measuring device (30) is configured to measure the input and output signals in synchrony with the heartbeat of the subject comprising the stent, preferably by using a transformed ECG signal as a trigger signal wherein this trigger signal is provided to the measuring device to launch the electromagnetic fields, more preferably, wherein the system further comprises means for obtaining the ECG of the subject, and wherein the measuring device is configured to receive the ECG signal from the means for obtaining the ECG.

12. The system according to any of the previous claims, wherein the antennas (10, 12) are co-polarized antennas.

13. The system according to any of the previous claims, wherein the antennas (10, 12) are linearly polarised at a frequency span that includes the average stent resonance frequency in biological tissue (0.7 GHz), preferably the span ranges between 0.1 and 3 GHz.

14. The system according to any of the previous claims, wherein $m_D$ comprises or consists of the representative frequency times the echo delay of the direct electromagnetic field (26), launched from an emitter antenna (10, 12) and directly collected by a receiver antenna (12, 10) different than the emitter antenna (10, 12), and ms comprises or consists of the representative frequency times the echo delay of the scattered electromagnetic field (22) component launched from an emitter antenna (10, 12), scattered by the stent (14) and collected by a receiver antenna (12, 10) different than the emitter antenna (10, 12), optionally wherein the parameter $m_S$ further comprises or is averaged with the representative frequency of the electromagnetic field launched from an emitter antenna (10, 12), reflected and/or scattered by the stent (14) and collected by the same antenna (10, 12).

15. The system according to any one of the previous claims wherein $m_S$ comprises a representative frequency and/or an echo delay associated to a simulation or *in vitro* measurements of different stent and/or lesion models, in addition to, or instead of the second scattered electromagnetic field (22) component.

Fig. 1

Fig. 2

Fig. 3

Fig. 4

Fig. 5

Fig. 6

|  | TIME DOMAIN | | | | FREQUENCY DOMAIN | | | |
|---|---|---|---|---|---|---|---|---|
|  | DIRECT | | SCATTERED | | DIRECT | | SCATTERED | |
|  | $t_0$ | std | $t_0$ | std | $f_0$ | std | $f_0$ | std |
| *DAY* | **C01** | | | | | | | |
| 0 | 1,89 | 0,01 | 2,69 | 0,03 | 1,31 | 0,01 | 1,09 | 0,03 |
| 7 | 1,74 | 0,00 | 2,69 | 0,01 | 1,62 | 0,00 | 1,18 | 0,01 |
| 14 | 1,78 | 0,01 | 2,90 | 0,02 | 1,58 | 0,01 | 1,07 | 0,02 |
| 29 | 1,74 | 0,01 | 2,69 | 0,02 | 1,58 | 0,01 | 1,17 | 0,02 |
| *DAY* | **C03** | | | | | | | |
| 0 | 2,04 | 0,01 | 3,30 | 0,01 | 1,33 | 0,01 | 1,25 | 0,01 |
| 7 | 1,38 | 0,56 | 2,66 | 0,03 | 1,57 | 0,56 | 1,07 | 0,03 |
| 14 | 1,80 | 0,01 | 2,89 | 0,04 | 1,41 | 0,01 | 1,07 | 0,04 |
| 29 | 1,71 | 0,01 | 2,70 | 0,02 | 1,63 | 0,01 | 1,10 | 0,02 |
| *DAY* | **C04** | | | | | | | |
| 0 | 1,96 | 0,01 | 3,32 | 0,02 | 1,59 | 0,01 | 1,28 | 0,02 |
| 8 | 1,71 | 0,01 | 2,63 | 0,02 | 1,58 | 0,01 | 1,09 | 0,02 |
| 15 | 1,72 | 0,01 | 2,76 | 0,03 | 1,51 | 0,01 | 1,06 | 0,03 |
| 29 | 1,80 | 0,01 | 2,93 | 0,04 | 1,45 | 0,01 | 1,08 | 0,04 |

|  | TIME DOMAIN | | | | FREQUENCY DOMAIN | | | |
|---|---|---|---|---|---|---|---|---|
|  | DIRECT | | SCATTERED | | DIRECT | | SCATTERED | |
|  | t0 | std | t0 | std | f0 | std | f0 | std |
| *DAY* | **C05** | | | | | | | |
| 0 | 1,73 | 0,01 | 2,62 | 0,03 | 1,32 | 0,01 | 1,11 | 0,03 |
| 7 | 1,66 | 0,01 | 2,57 | 0,03 | 1,52 | 0,01 | 1,02 | 0,03 |
| 14 | 1,72 | 0,01 | 2,63 | 0,02 | 1,57 | 0,01 | 1,05 | 0,02 |
| 32 | 1,77 | 0,01 | 2,69 | 0,02 | 1,53 | 0,01 | 1,14 | 0,02 |
| *DAY* | **C06** | | | | | | | |
| 0 | 1,78 | 0,01 | 2,73 | 0,02 | 1,43 | 0,01 | 1,06 | 0,02 |
| 7 | 1,92 | 0,02 | 2,85 | 0,03 | 1,31 | 0,02 | 1,04 | 0,03 |
| 14 | 1,91 | 0,01 | 2,91 | 0,03 | 1,43 | 0,01 | 1,12 | 0,03 |
| 26 | 1,87 | 0,01 | 2,95 | 0,02 | 1,51 | 0,01 | 1,11 | 0,02 |

Fig. 7

EP 4 699 527 A1

**Fig. 8**

*** Presumed 90% obstruction in proximal stent section

Europäisches Patentamt

European Patent Office

Office européen des brevets

# EUROPEAN SEARCH REPORT

**Application Number**

EP 24 38 2920

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GONZÁLEZ-LÓPEZ GISELLE ET AL: "Resonance-Based Microwave Technique for Body Implant Sensing", SENSORS, vol. 19, no. 22, 6 November 2019 (2019-11-06), page 4828, XP093219247, CH ISSN: 1424-8220, DOI: 10.3390/s19224828 * page 1, paragraph 2; page 2, paragraph 3; page 4, paragraph 3; page 5, paragraph 2; page 6, paragraphs 1-4; figures 3,11,12 * | 1-15 | INV. A61B5/05 A61B5/0507 A61B5/06 A61B8/00 A61F2/82 ADD. H01P3/02 |
| A | US 2016/213321 A1 (BERNSTEIN ASSAF [IL] ET AL) 28 July 2016 (2016-07-28) * claim 67 * | 11 | |
| A | GÁLVEZ-MONTÓN CAROLINA ET AL: "Ex vivo assessment and in vivo validation of non-invasive stent monitoring techniques based on microwave spectrometry", SCIENTIFIC REPORTS, [Online] vol. 8, no. 1, 4 October 2018 (2018-10-04), XP093219249, US ISSN: 2045-2322, DOI: 10.1038/s41598-018-33254-9 Retrieved from the Internet: URL:https://www.nature.com/articles/s41598-018-33254-9> [retrieved on 2024-10-29] * figures 1,5; table 1 * | 1-15 | TECHNICAL FIELDS SEARCHED (IPC) A61B A61F H01P |

-/--

The present search report has been drawn up for all claims

| Place of search | Date of completion of the search | Examiner |
|---|---|---|
| The Hague | 5 November 2024 | Chacon Caldera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 2**

Europäisches
Patentamt

European
Patent Office

Office européen
des brevets

## EUROPEAN SEARCH REPORT

**Application Number**

EP 24 38 2920

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | AMOROS GARCIA DE ET AL: "Microwave spectrometry for the evaluation of coronary stents", EUROPEAN HEART JOURNAL, vol. 41, no. Supplement_2, 1 September 2020 (2020-09-01), XP093219251, GB ISSN: 0195-668X, DOI: 10.1093/ehjci/ehaa946.2530 * abstract * | 1-15 | |
| A | WO 2019/057685 A1 (UNIV BARCELONA [ES]; UNIV CATALUNYA POLITECNICA [ES] ET AL.) 28 March 2019 (2019-03-28) * figure 9 * | 1-15 | |
| A | Gianluca Arauz Garofalo: "Prospects of microwave spectrometry for vascular stent monitoring", , 13 June 2017 (2017-06-13), pages 1-126, XP055456123, Spain Retrieved from the Internet: URL:http://www.tdx.cat/bitstream/handle/10 803/404376/GAG_PhD_THESIS.pdf?sequence=1&i sAllowed=y [retrieved on 2018-03-02] * figure 2.1 * | 1-15 | TECHNICAL FIELDS SEARCHED (IPC) |

The present search report has been drawn up for all claims

| Place of search | Date of completion of the search | Examiner |
|---|---|---|
| The Hague | 5 November 2024 | Chacon Caldera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 24 38 2920

This annex lists the patent family members relating to the patent documents cited in the above-mentioned European search report.
The members are as contained in the European Patent Office EDP file on
The European Patent Office is in no way liable for these particulars which are merely given for the purpose of information.

05-11-2024

| Patent document cited in search report | Publication date | Patent family member(s) | | Publication date |
|---|---|---|---|---|
| US 2016213321 A1 | 28-07-2016 | US | 2009299175 A1 | 03-12-2009 |
| | | US | 2013123614 A1 | 16-05-2013 |
| | | US | 2015335310 A1 | 26-11-2015 |
| | | US | 2016213321 A1 | 28-07-2016 |
| WO 2019057685 A1 | 28-03-2019 | EP | 3684252 A1 | 29-07-2020 |
| | | EP | 4378384 A2 | 05-06-2024 |
| | | HR | P20240539 T1 | 19-07-2024 |
| | | HU | E067105 T2 | 28-09-2024 |
| | | PL | 3684252 T3 | 29-07-2024 |
| | | RS | 65552 B1 | 28-06-2024 |
| | | WO | 2019057685 A1 | 28-03-2019 |

EPO FORM P0459

For more details about this annex : see Official Journal of the European Patent Office, No. 12/82